# EUROPEAN PATENT APPLICATION

(11) **EP 4 501 264 A1**
(43) Date of publication of application: **05.02.2025**
(21) Application number: 23871063.6
(22) Date of filing: 28.09.2023
(51) Int. Cl.: A61B 18/20, A61B 18/18

(54) **OPTICAL DEPILATOR**

(30) Priority: 30.09.2022 CN 202222651803 U; 30.09.2022 CN 202222651333 U
(71) Applicant: One Beauty Technology Co., Ltd., Shenzhen, Guangdong 518000 (CN)
(72) Inventor: PAN, Ninghua, Shenzhen, Guangdong 518000 (CN)
(74) Representative: Studio Torta S.p.A.
(86) International application number: PCT/CN2023/122789
(87) International publication number: WO 2024/067839

(57) **Abstract**

Provided is an optical depilator, which comprises a main body and an optical depilation assembly. The main body comprises a housing and a control mainboard arranged inside the housing. The optical depilation assembly can be electrically connected to the control mainboard inside the main body. The control mainboard is used for providing control signals and a working current for the optical depilation assembly. The optical depilation assembly comprises a shell and a light-emitting assembly and a heat dissipation apparatus arranged in the shell. The shell is separated from the housing. The shell is provided with a light outlet, and the light-emitting assembly and the light outlet are oppositely arranged. A heat dissipation channel is formed in the shell, and the light-emitting assembly and the heat dissipation apparatus are arranged in the heat dissipation channel.

## Description

### CROSS-REFERENCE TO RELATED APPLICATIONS

This application claims priorities to a Chinese patent application No. 202222651803.6, titled "Optical Hair Removal Device", filed on September 30, 2022, and a Chinese patent application No. 202222651333.3, titled "Optical Hair Removal Device System and its Optical Hair Removal Device", filed on September 30, 2022, all of which are incorporated into this application by reference.

### TECHNICAL FIELD

The present application relates to the technical field of hair removal devices, in particular to an optical hair removal device.

### BACKGROUND

The hair removal device is becoming more and more popular as a kind of beauty device that can remove body hair on the surface of skin, and people's requirements are getting higher and higher, that is, for different parts of hair on the surface of the skin, the hair removal device has different requirements on the function, a caliber of the light outlet, etc. The existing hair removal device mostly adopts the hair removal assembly and the main body that are formed integratedly, which has a single function and cannot meet the user's requirements, and reduces the user's sense of experience. If other functions need to be realized, a different hair removal device needs to be bought, the general applicability is not high, increasing the economic burden of the user.

Therefore, the existing technology needs to be improved and enhanced.

### SUMMARY

The present application provides an optical hair removal device that enables an optical hair removal assembly to be separated from a main body.

A first aspect of the present application provides an optical hair removal device, which includes:
a main body, including a housing and a control main board provided inside the housing;
an optical hair removal assembly, electrically connectable to the control main board within the main body, the control main board is configured to provide control signals and working currents for the optical hair removal assembly; and
the optical hair removal assembly includes a housing body, a light emitting assembly and a heat dissipation device provided within the housing;
the housing body is separated from the housing, the housing is provided with a light outlet, the light emitting assembly is opposite to the light outlet, and the heat dissipation channel is formed inside the housing; and
the light emitting assembly and the heat dissipation device are provided inside the heat dissipation channel.

In one embodiment, the optical hair removal device includes a detachable assembly, the main body and the optical hair removal assembly are detachably connected by the detachable assembly.

In one embodiment, the main body is snapped to the optical hair removal assembly by the detachable assembly;
the detachable assembly includes a snapping body and a snapping base for snap-fit, and the snapping body is provided in one of the main body and the optical hair removal assembly; and
the snapping base is provided in another one of the main body and the optical hair removal assembly.

In one embodiment, the snapping body is protruded at a rear end of the housing, and the snapping body includes a convex extension and a snapping portion connected to each other, an angle is formed between the snapping portion and the convex extension, and the convex extension is connected to the rear end of the housing; and
the snapping base is provided at a front end of the main body, the snapping base is provided with a groove with an opening forward, and the snapping portion is snapped in the groove when the optical hair removal assembly is docked with the main body.

In one embodiment, the detachable assembly further includes a limitation member, the limitation member is provided in one of the main body and the optical hair removal assembly, and another one of the main body and the optical hair removal assembly is provided with a limitation groove corresponding one-in-one to the limitation member; and
the limitation member is movably accessed into the limitation groove, to lock the optical hair removal assembly and the main body.

In one embodiment, the limitation member is provided at a front end of the main body, the limitation member includes a pressing portion and a limitation portion, and the pressing portion is protruded at one of sides of the main body, and the limitation portion is protruded at a front end of the main body;
the limitation groove is provided at a rear end of the optical hair removal assembly; and
the limitation member is movably accessed into the limitation groove, to lock the optical hair removal assembly and the main body.

In one embodiment, the detachable assembly is a connection cable, and the connection cable is detachably connected to at least one of the optical hair removal assembly and the main body; and
an end of the connection cable is electrically connected to the optical hair removal assembly, another end of the connection cable is electrically connected to the main body, and a control main board of the main body is electrically connected to the optical hair removal assembly by the connection cable.

In one embodiment, the main body is electrically connected to the optical output assembly by a converting device, and the converting device comprises a converting wire and at least one converter;
each converter is detachably and electrically connected to one of the main body and the optical hair removal assembly by the detachable assembly, an end of the converting wire is physically and electrically connected to the converter, and another end of the converting wire is physically and electrically connected to another one of the main body and the optical hair removal assembly.

In one embodiment, the converter is snapped to the main body or the optical hair removal assembly; and
a set of electrical connectors adapted and electrically connected to each other are provided at surfaces where the converter is docked with the main body or the optical hair removal assembly, respectively.

In one embodiment, the converting device includes two converters provided at two opposite ends of the converting wire, and the two converters are a first converter and a second converter, respectively;
the first converter is snapped to the main body by the detachable assembly, a first set of electrical connectors adapted and electrically connected to each other are provided at surfaces where the first converter is docked with the main body, respectively; and
the second converter is snapped to the optical hair removal assembly by the detachable assembly, and a second set of electrical connectors adapted and electrically connected to each other are provided at surfaces where the second converter is docked with the optical hair removal assembly.

In one embodiment, the converting device includes a converting wire and a converter, an end of the converting wire is physically and electrically connected to the converter, and another end of the converting wire is physically and electrically connected to the main body, wherein the converter is snapped to the optical hair removal assembly by the detachable assembly; and
a set of electrical connectors adapted and electrically connected to each other are provided at surfaces where the converter is docked with the optical hair removal assembly.

In one embodiment, the converting device includes a converting wire and a converter, an end of the converting wire is physically and electrically connected to the converter, and another end of the converting wire is physically and electrically connected to the optical hair removal assembly, and the converter is snapped to the main body by the detachable assembly; and
a set of electrical connectors adapted and electrically connected to each other are provided at surfaces where the converter is docked with the main body.

In one embodiment, the optical hair removal device includes a plug-in connection terminal set;
the plug-in connection terminal set comprises a first connection terminal and a second connection terminal, the first connection terminal is provided at a rear end of the optical hair removal assembly, the second connection terminal is provided at a front end of the main body, and the first connection terminal is pluggable with the second connection terminal.

In one embodiment, the hair removal device includes a power transmission cable, an end of the power transmission cable is fixed to the optical hair removal assembly, and an electrical connection of the power transmission cable and the optical hair removal assembly is kept, another end of the power transmission cable is fixed to the main body, and an electrical connection of the power transmission cable and the main body is kept.

In one embodiment, the optical hair removal device further includes a plurality of the main bodies, each main body is provided with a battery inside, and the battery is electrically connected to the control main board.

In one embodiment, the optical hair removal device further includes a plurality of the optical hair removal assemblies, the plurality of the optical hair removal assemblies include a first optical hair removal assembly and a second optical hair removal assembly, a light outlet of the first optical hair removal assembly is a circular light outlet, and a light outlet of the second optical hair removal assembly is a square light outlet.

In one embodiment, the optical hair removal device further includes an adapter, the adapter is separated from the main body, and the adapter and the main body are electrically connected to the control main board by a wire.

In one embodiment, the main body includes a housing;
an end of the heat dissipation channel is an air inlet and another end of the heat dissipation channel is an air outlet, and the air inlet is located on a side of the housing and the air outlet is located on another side of the housing.

In one embodiment, the light emitting assembly includes a light source, a reflective cover, and a lens, the light source is fixed on an inner side of the reflective cover, and is a pulsed lamp or a laser light source;
the housing is also provided with a positioning bracket, and the reflective cover is fixed to the positioning bracket; and
the lens is provided at a front of the reflective cover for converging light emitted by the light source.

Compared to the related art, in the optical hair removal device provided in the present application, the housing body of the optical hair removal assembly is separated from the housing of the main body, and the optical hair removal assembly can be separated from the main body, which is convenient for the replacement of the optical hair removal assembly, so that the user can select the optical hair removal assembly with different functions according to his or her own needs, and improve the user's experience. Moreover, the optical hair removal assembly is provided with a heat dissipation channel and a heat dissipation device, so that the optical hair removal assembly can be used to avoid the accumulation of a large amount of heat, which may result in burns and other dangers.

### BRIEF DESCRIPTION OF THE DRAWINGS

FIG. 1 is a three-dimensional structure view of the optical hair removal device in the present embodiment.
FIG. 2 is a sectional structure view of the optical hair removal device in the present embodiment.
FIG. 3 is a three-dimensional structure view of the main body in the present embodiment.
FIG. 4 is a sectional structure view of the main body in the present embodiment.
FIG. 5 is a sectional structure view of the optical hair removal assembly in the present embodiment.
FIG. 6 is a three-dimensional structure view of the optical hair removal assembly in the present embodiment.
FIG. 7 is another three-dimensional structure view of the optical hair removal assembly in the present embodiment.
FIG. 8 is the three-dimensional structure view of the optical hair removal assembly in other embodiments.
FIG. 9 is the three-dimensional structure view of the optical hair removal device in other embodiments.
FIG. 10 is the three-dimensional structure view of the optical hair removal device in one embodiment.
FIG. 11 is the three-dimensional structure view of the optical hair removal device in another embodiment.
FIG. 12 is the three-dimensional structure view of the optical hair removal device in one embodiment.
FIG. 13 is the three-dimensional structure view of the optical hair removal device in another embodiment.

### DETAILED DESCRIPTION OF THE EMBODIMENTS

The present application provides an optical hair removal device, and in order to make the purpose, technical solution and effect of the present application clearer and more explicit, the present application is described in further detail hereinafter with reference to the accompanying drawings and by way of embodiments. It should be understood that the specific embodiments described herein are only for explaining the present application and are not intended to limit the present application.

The present application provides an optical hair removal device.

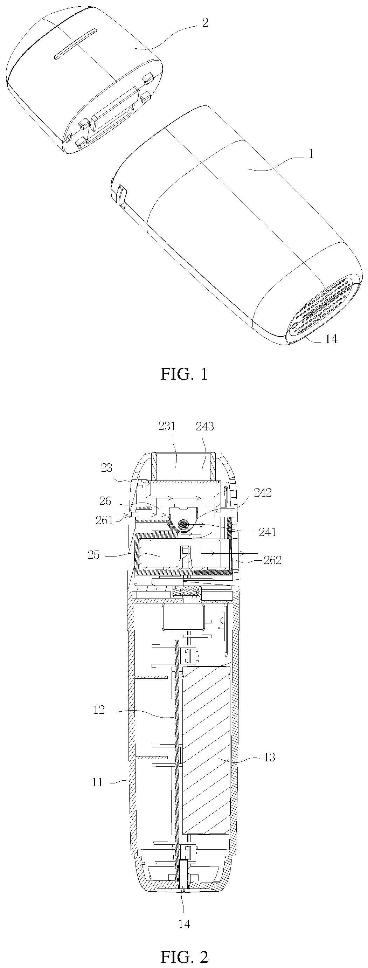

Referring to FIG. 1, FIG. 1 is a three-dimensional structure view of the optical hair removal device in the present embodiment. For convenience of description, it is provided that the optical hair removal device has a light emission direction in front and, conversely, another direction in the back.

The optical hair removal device of the present embodiment includes a main body 1 and an optical hair removal assembly 2. The main body 1 includes a housing 11 and the optical hair removal assembly 2 includes a housing body 21. The housing 11 of the main body 1 is separated from the housing body 21 of the optical hair removal assembly 2, so that the main body 1 and the optical hair removal assembly 2 can be separated, to facilitate the replacement of the optical hair removal assembly 2.

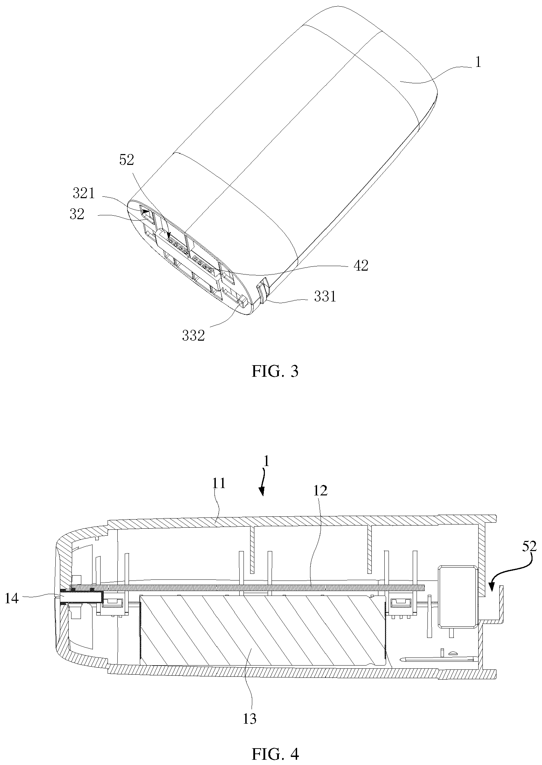

Referring to FIGS. 2 to 4, FIG. 2 is a sectional structure view of the optical hair removal device in this embodiment. FIG. 3 is a three-dimensional structure view of the main body 1 in this embodiment. FIG. 4 is a sectional structure view of the main body 1 in this embodiment. The main body 1 includes a housing and a control main board 12 provided inside the housing. In this embodiment, the control main board 12 is a printed circuit board.

The main body 1 is also provided with a battery 13, and the housing 11 is provided with a holding cavity, and the battery 13 is located in the holding cavity. The battery 13 is electrically connected to the control board 12 to power the entire optical hair removal device. Specifically, the battery 13 may be a lithium battery 13. Then when the power input terminal 14 is connected to a power source, the control main board 12 may control charging of the battery 13.

Further, the rear end of the main body 1 is provided with a power input terminal 14, the power input terminal 14 is electrically connected to the control main board 12, and the control main board 12 controls the power to be input. Since the battery 13 is electrically connected to the control main board 12, the control main board 12 can control charging of the battery 13 when the power input terminal 14 is connected to a power source.

In other embodiments, there are a plurality of main bodies 1, each main body is provided with the battery 13, and when the battery 13 of the main body 1 is depleted, the battery 13 can be replaced immediately with another main body 1 with a full charged battery 13, so that the plurality of main bodies 1 can be used alternately, thereby increasing the length of time for continuous use of the optical hair removal device.

The optical hair removal device also includes an adapter (not shown). The adapter and the main body 1 are provided separately from each other. The adapter and the main body 1 are electrically connected to the control main board 12 by wires. The power input terminal 14 can be directly connected to the power source, and after the adapter is adapted to regulate the voltage, the optical hair removal device is directly powered. The adapter is provided separately from the main body 1, so that when the optical hair removal device is used, there is no need to hold the adapter to increase the weight, so that the hair removal operation is more lightweight.

Specifically, the wire includes a first wire and a second wire provided at two ends of the adapter. One end of the first wire is electrically connected to the adapter and another end of the first wire is provided with a plug to electrically connect to a fixed socket. One end of the second wire is electrically connected to the adapter and another end of the second wire is electrically connected to the main body 1. Specifically, since the power input terminal 14 is electrically connected to the control main board 12, and the control main board 12 is electrically connected to the battery 13, the control main board 12 can control charging of the battery 13 when the adapter and the wire are energized.

The power input terminal 14 is electrically connected to the control main board 12, and another end of the second wire can be electrically connected to the control main board 12 by its' electrical connection to the power input terminal 14. At this time, the adapter and the power input terminal 14 can not only charge the optical hair removal device containing the battery 13, but also directly electrically connect and energize the optical hair removal device, i.e., even if the main body 1 is not provided with the battery 13 or the battery 13 is damaged, the adapter and the power input terminal 14 can cooperate to power the optical hair removal device to enable the optical hair removal to be continued to be used.

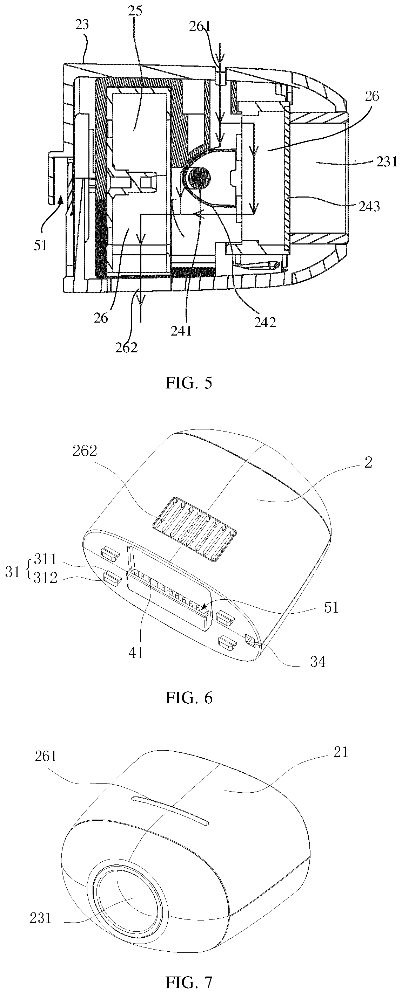

Referring to FIGS. 5 and 6, FIG. 5 is a cross-sectional structure view of the optical hair removal assembly 2 in this embodiment, and FIG. 6 is a the three-dimensional structure view of the optical hair removal assembly 2 in this embodiment. The optical hair removal assembly 2 is used to output a strong light to remove hair from the skin. The optical hair removal assembly 2 is provided separately from the main body 1. The optical hair removal assembly 2 includes a housing body 23, a light emitting assembly, and a heat dissipation device 25. The weight of the optical hair removal assembly 2 is light than the weight of the main body 1.

The volume of the housing body 23 is smaller than the volume of the housing 11. The housing body 23 is used to accommodate the light emitting assembly and the heat dissipation device 25. The housing body 23 is provided separately from the housing 11, and a rear end of the housing body 23 can be detachably connected to the main body 1, and a light outlet 231 is provided at a front end of the housing body 23.

The lens 243 can not only seal the light outlet 231, but can also be used to converge the light emitted by the light source 241 to achieve a better hair removal effect. In this embodiment, the shape of the lens 243 may be round or square, which is determined specifically based on actual needs.

A positioning bracket is also provided in the housing body 23, and the reflective cover 242 is fixed to the positioning bracket.

A heat dissipation channel 26 is formed in the housing body 23. The heat dissipation channel 26 is provided with an air inlet 261 and an air outlet 262, i.e., an end of the heat dissipation channel 26 is the air inlet 261 and another end of the heat dissipation channel 26 is the air outlet 262. Specifically, the air inlet 261 is provided on a side of the housing body 23, and the air outlet 262 is provided on another side of the housing body 23.

The heat dissipation device 25 is provided within the heat dissipation channel 26. The heat dissipation device 25 may be a cooling fan.

The heat dissipation device 25 generates negative pressure to attract cold air in from the air inlet 261, the cold air enters into the heat dissipation channel 26 to carry the heat inside the heat dissipation channel 26, and be discharged from the air outlet 262, so as to maintain the temperature of the optical hair removal assembly 2.

The light emitting assembly is located in the heat dissipation channel 26. Since the light emitting assembly needs to emit high-frequency strong light, which generates a large amount of heat, and the heat dissipation channel 26 is able to dissipate the heat of the light emitting assembly.

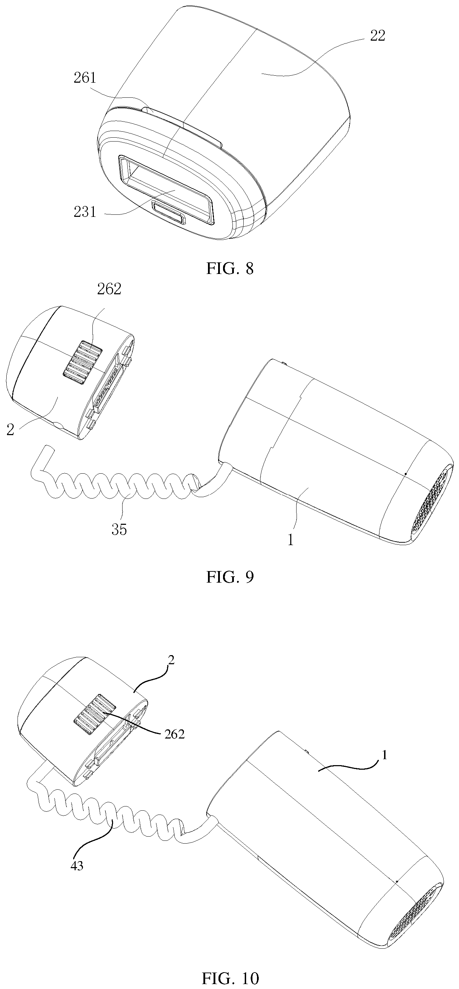

Referring to FIGS. 7 and 8, FIG. 7 is another three-dimensional structure view of the optical hair removal assembly 2 in this embodiment, and FIG. 8 is a three-dimensional structure view of the optical hair removal assembly 2 in other embodiments. There may be a plurality of optical hair removal assemblies 2, and since the housing 11 of the main body 1 and the housing body 21 of the optical hair removal assembly 2 are separated, the main body 1 and the optical hair removal assembly 2 can be separated, and at this time, the user can replace the optical hair removal assembly 2 as needed.

The plurality of optical hair removal assemblies 2 includes a first optical hair removal assembly 21 and a second optical hair removal assembly 22. The light outlet 231 of the first optical hair removal assembly 21 is of circular shape, as shown in FIG. 7. The light outlet 231 of the second optical hair removal assembly 22 is of square shape, as shown in FIG. 8. Correspondingly, the lens 243 may be a round lens or a square lens. Different shapes of the light outlet 231 have different areas. For example, for a skin region with a large mount of hairs, the optical hair removal assembly 2 may be selected with a larger area of the light outlet 231.

The powers of the first optical hair removal assembly 21 and the second optical hair removal assembly 22 may be different, and the power is specifically provided according to the human skin region to which the optical hair removal assembly 2 is applied.

The optical hair removal device includes a detachable assembly, and the optical hair removal assembly 2 and the main body 1 are detachably connected by the detachable assembly, and it is convenient for the user to select different optical hair removal assemblies 2 and the main body 1 according to his or her own needs, which is convenient to disassemble and replace the optical hair removal assemblies 2, thus it is very convenient and simple to operate.

Continuing to refer to FIGS. 3 and 6, the main body 1 and the optical hair removal assembly 2 are connected by the detachable assembly.

The detachable assembly includes a snapping body 31 and a snapping seat 32 for snap-fit. The snapping body 31 is provided in one of the main body 1 and the optical hair removal assembly 2, and the snapping seat 32 is provided in another one of the main body 1 and the optical hair removal assembly 2. Specifically, the snapping body 31 is protruded at a rear end of the housing 11 of the optical hair removal assembly 2, and the snapping seat 32 is provided at a front end of the main body 1. The snapping body 31 includes a convex extension 311 and a snapping portion 312 connected to each other. An angle is formed between the snapping portion 312 and the convex extension 311, and the convex extension 311 is connected to a rear end of the housing body 23. The angle may be an acute or right angle. The snapping seat 32 is provided with a groove 321 with an opening forward, and the shape of the groove 321 corresponds to that of the snapping body 31, so that when the optical hair removal assembly 2 is docked with the main body 1, the snapping portion 312 is snapped in the groove 321 to realize the snapping connection of the optical hair removal assembly 2 and the main body 1.

In other embodiments, the snapping body 31 is provided at a front end of the main body 1, and the snapping seat 32 is provided at a rear end of the optical hair removal assembly 2.

In this embodiment, there are four snapping bodies 31 and they are distributed in a matrix at the rear end of the optical hair removal assembly 2. Similarly, four grooves 321 are distributed in a matrix at the front end of the main body 1.

The detachable assembly further includes a limitation member provided in one of the main body 1 and the optical hair removal assembly 2, and another one of the main body 1 and the optical hair removal assembly 2 is provided with a limitation groove 34 corresponding to the limitation member, and the limitation member can be movably accessed into the limitation groove 34, to lock the optical hair removal assembly 2 and the main body 1.

Specifically, the limitation member is provided at the front end of the main body 1, and the limitation groove 34 is provided at the rear end of the optical hair removal assembly 2. The limitation member includes a pressing portion 331 and a limitation portion 332, the pressing portion 331 is protruded from one of the sides of the main body 1, and the limitation portion 332 is protruded from the front end of the main body 1. The limitation portion 332 can be movably accessed into the limitation groove 34, to lock the optical hair removal assembly 2 and the main body 1.

Referring to FIG. 9, FIG. 9 is a three-dimensional structure view of the optical hair removal device in other embodiments. In this embodiment, the detachable assembly is a connection cable 35, the connection cable 35 is detachably connected to at least one of the optical hair removal assembly 2 and the main body 1, and an end of the connection cable 35 is electrically connected to the optical hair removal assembly 2 and another end of the connection cable 35 is electrically connected to the main body 1, and the control main board 12 of the main body 1 is electrically connected to the optical hair removal assembly 2 via the connection cable 35. At this time, the main body 1 is separated from the optical hair removal assembly 2, and when in use, only the optical hair removal assembly 2 needs to be held. Therefore, the above optical hair removal device can avoid holding the whole larger and heavier hair removal device for a long time. On the one hand, the weight of the optical hair removal assembly 2 is small, which can reduce the weight, and on the other hand, the volume of the optical hair removal assembly 2 is small, which can be operated flexibly. Moreover, the connection cable 35 is at least partially in spiral shape and has elasticity, so that it can be stretched when subjected to an external force to reduce the limitation of the length of the connection cable 35 when the optical removal device is in use, to be used conveniently, and it can be naturally retracted to be stored conveniently when the external force is removed.

Continuing to refer to FIGS. 1, 3, and 6, in this embodiment, when the optical hair removal assembly 2 is connected to the main body 1, the optical hair removal assembly 2 can be electrically connected to the control main board 12 within the housing 11 of the main body 1. Specifically, the optical hair removal device includes a plug-in connection terminal set, and the optical hair removal assembly 2 is electrically connected to the control main board 12 within the main body 1 via the plug-in connection terminal set, at this time, the control main board 12 can provide a control signal and a working current for the optical hair removal assembly 2 to realize the work of the optical hair removal assembly 2. The plug-in connection terminal set includes a first connection terminal 41 and a second connection terminal 42, the first connection terminal 41 is provided at a rear end of the optical hair removal assembly 2, and the second connection terminal 42 is provided at a front end of the main body 1. In other embodiments, the first connection terminal 41 may also be provided at the front end of the main body 1, and the second connection terminal 42 is provided correspondingly at the rear end of the optical hair removal assembly 2.

In order to protect the connection terminal set, in this embodiment, the rear end of the optical hair removal assembly 2 is provided with a first protection groove 51, and the front end of the main body 1 is provided with a second protection groove 52. Openings of the first protection groove 51 and the second protection groove 52 are opposite to each other. The first connection terminal 41 is provided in the first protection groove 51, and the second connection terminal 42 is provided in the second protection groove 52. When the optical hair removal assembly 2 is docked with the main body 1, the first protection groove 51 is docked with the second protection groove 52, so that the second connection terminal 42 is plugged with the first connection terminal 41, and the optical hair removal assembly 2 is electrically connected to the control main board 12 in the main body 1.

Referring to FIG. 10, FIG. 10 is a three-dimensional structure view of the optical hair removal device in an embodiment. In this embodiment, the hair removal device includes a power transmission cable 43 for realizing the electrical connection of the main body 1 and the optical hair removal assembly 2. Specifically, an end of the power transmission cable 43 is fixed to the optical hair removal assembly 2, and an electrical connection of the power transmission cable 43 and the optical hair removal assembly 2 is kept, and another end of the power transmission cable 43 is fixed to the main body 1 and an electrical connection of the power transmission cable 43 and the main body 1 is kept. In this embodiment, the main body 1 and the optical hair removal assembly 2 may be connected and fixed by the detachable assembly to form a whole, or the main body 1 and the optical hair removal assembly 2 are separated, and when in use, only the optical hair removal assembly 2 needs to be held, which can avoid holding the whole larger and heavier hair removal device for a long time. On the one hand, the weight of the optical hair removal assembly 2 is small, which can reduce the weight, and on the other hand, the volume of the optical hair removal assembly 2 is small, which can be operated flexibly. Moreover, the power transmission cable 43 is at least partially in spiral shape and has elasticity, so that it can be stretched when subjected to an external force to reduce the limitation of the length of the power transmission cable 43 when the optical removal device is used, to be used conveniently, and it can be naturally retracted to be stored conveniently when the external force is removed.

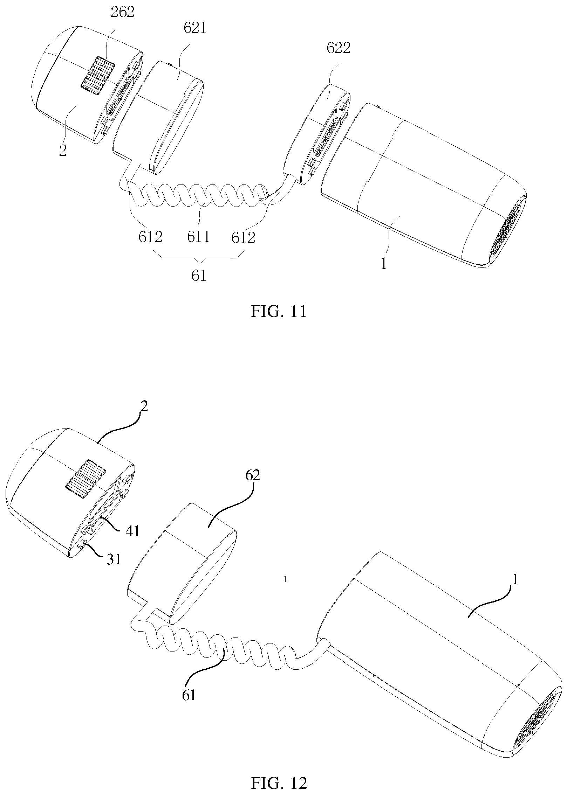

Referring to FIG. 11, FIG. 11 is a three-dimensional structure view of the optical hair removal device in another embodiment. The main body 1 and the optical hair removal assembly 2 are electrically connected by a converting device, and the main body 1 and the optical hair removal assembly 2 can be detachably connected by the converting device, so that the optical hair removal assembly 2 can be separated from the main body 1, and when in use, only the optical hair removal assembly 2 needs to be held. Therefore, the above optical hair removal device can avoid holding the whole larger and heavier hair removal device for a long time. On the one hand, the weight of the optical hair removal assembly 2 is small, which can reduce the weight, and on the other hand, the volume of the optical hair removal assembly 2 is small, which can be operated flexibly.

The converting device includes a converting wire 61 and at least one converter 62. the converter 62 is detachably and electrically connected to one of the main body 1 and the optical hair removal assembly 2 by the detachable assembly, one end of the converting wire 61 is physically and electrically connected to the converter 62, and another end of the converting wire 61 is physically and electrically connected to another one of the main body 1 and the optical hair removal assembly 2. The physical connection herein may be a removable connection or a fixed connection in the form of a plug-in connection, etc.

The converter 62 is snapped to the main body 1 or the optical hair removal assembly 2. A set of electrical connectors adapted and electrically connected to each other, respectively, are provided at surfaces where the converter 62 is docked with the main body 1 or the optical hair removal assembly 2.

Specifically, the converting device includes two converters 62 provided at two opposite ends of the converting wire 61, and the two converters 62 are a first converter 621 and a second converter 622, respectively.

The converting wire 61 includes an elastic portion 611 and a connection portion 612 provided at both ends of the elastic portion 611. The elastic portion 611 can be stretched when subjected to an external force to reduce the limitation of the length of the converting wire 61 when the optical hair removal device is used, to be used conveniently, and can be naturally retracted be stored conveniently when the external force is removed. The two connection portions 612 are connected to the first converter 621 and the second converter 622, respectively.

The first converter 621 is snapped to the main body 1 by the detachable assembly. The second converter 622 is snapped to the optical hair removal assembly 2 by the detachable assembly. The detachable assembly herein may be the snapping body 31 and the snapping base 32 or a connection cable 35 for snap-fit as described above.

Moreover, a first set of electrical connectors adapted and electrically connected to each other are provided at surfaces where the first converter 621 is docked with the main body 1. A second set of electrical connectors adapted and electrically connected to each other are provided at the surfaces where the second converter 622 is docked with the optical hair removal assembly 2. The first set of electrical connectors and the second set of electrical connectors herein may be the plug-in connection terminal set as described above.

Referring to FIG. 12, FIG. 12 is a three-dimensional structure view of the optical hair removal device in one of the embodiments. The converting device includes a converting wire 61 and a converter 62. Specifically, an end of the converting wire 61 is physically connected to the converter 62 and their electrical connection kept, and another end of the converting wire 61 is physically connected to the main body 1 and their electrical connection is kept. The physical connection herein may be a removable connection or a fixed connection in the form of a plug-in connection, etc.

The converter 62 is detachably connected to the optical hair removal assembly 2 by the detachable assembly. Moreover, a plug-in connection terminal set is provided at surfaces where the converter 62 is docked with the optical hair removal assembly 2, respectively, to realize the electrical connection of the converter 62 and the optical hair removal assembly 2.

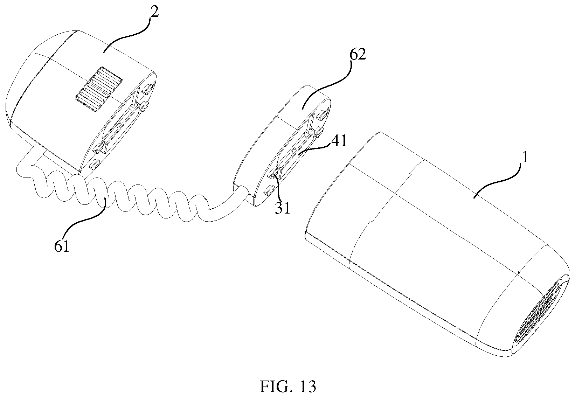

Referring to FIG. 13, FIG. 13 is a three-dimensional structure view of the optical hair removal device in another embodiment. The converting device includes a converting wire 61 and a converter 62. Specifically, an end of the converting wire 61 is physically connected to the converter 62 and their electrical connection is kept, and another end of the converting wire 61 is physically connected to the main body 1 and their electrical connection is kept. The physical connection herein may be a removable connection or a fixed connection in the form of a plug-in connection, etc.

The converter 62 is detachably connected to the main body 1 by the detachable assembly. Moreover, a plug-in connection terminal set is provided at the surface swhere the converter is docked with the main body 1, respectively, to realize the electrical connection of the converter 62 and the main body 1.

Therefore, the optical hair removal device of the present application has at least the following beneficial effects:

First, in the optical hair removal device provided in the present application, the optical hair removal assembly and the main body are detachably connected by the detachable assembly, and the optical hair removal assembly and the main body can be detached, which facilitates the replacement of the optical hair removal assembly, and enables the user to select the optical hair removal assembly with different functions according to his or her own needs, thereby improving the sense of experience of the user. Moreover, the optical hair removal assembly is provided with a heat dissipation channel and a heat dissipation device, so that the optical hair removal assembly can be used to avoid the accumulation of a large amount of heat, because the large amount of heat may result in scalding and other dangers.

Secondly, due to the installation of the main body with heavy components such as the main board and the battery, the optical hair removal assembly can be detachably and electrically connected to the main body through the converting device, and the optical hair removal assembly can be separated from the main body, so that the user only needs to hold the optical hair removal assembly when in use, thereby avoiding holding the whole larger and heavier main body for a long time. On the one hand, the weight of the optical hair removal assembly is small, which can reduce the weight, and on the other hand, the volume of the optical hair removal assembly is small, which can be operated flexibly.

Moreover, the converting device includes a converting wire and at least one converter, the converter is detachably and electrically connected to one of the main body and the optical hair removal assembly by the detachable assembly, and the converting wire is physically and electrically connected to another one of the main body and the optical hair removal assembly. The optical hair removal assembly and the main body can be detached and replaced, and the operation is very convenient and simple. The optical hair removal device includes a plurality of optical hair removal assemblies with different power and different shapes of light outlets, so that the user can select a suitable optical hair removal assembly according to his or her own usage needs. Therefore, the optical hair removal device can be used in a variety of ways to meet various needs, which prevents users from repeatedly purchasing hair removal devices with different functions, resulting in a waste of resources.

Moreover, the converting wire in the converting device includes an elastic portion, which can be stretched when subjected to an external force to reduce the limitation of the length of the converting wire when the optical hair removal device is used, to be used conveniently, and can be naturally retracted to be stored conveniently when the external force is removed.

Furthermore, a battery is provided in the main body. When the optical hair removal device is used, it cannot be limited by the location of the fixed socket, and can be used by the user anytime and anywhere, which is very convenient. Moreover, a plurality of main bodies may also be provided, each main body is provided with a battery. Since the main body is detachably connected to the optical hair removal assembly, when the battery power of one main body is depleted, it can be immediately replaced with another main body with a full charged battery , then the optical hair removal device can continue to be used.

It is understood that to those skilled in the art, equivalent substitutions or changes may be made in accordance with the technical solution of the present application and its inventive concept, and all such changes or substitutions shall fall within the scope of the claims appended to the present application.

## Claims

1. An optical hair removal device, **characterized by** comprising:
a main body, comprising a housing and a control main board provided inside the housing;
an optical hair removal assembly, electrically connectable to the control main board within the main body, wherein the control main board is configured to provide control signals and working currents for the optical hair removal assembly; and
wherein the optical hair removal assembly comprises a housing body, a light emitting assembly and a heat dissipation device provided within the housing;
the housing body is separated from the housing, the housing is provided with a light outlet, the light emitting assembly is opposite to the light outlet, and the heat dissipation channel is formed inside the housing; and
the light emitting assembly and the heat dissipation device are provided inside the heat dissipation channel.

2. The optical hair removal device according to claim 1, wherein the optical hair removal device comprises a detachable assembly, the main body and the optical hair removal assembly are detachably connected by the detachable assembly.

3. The optical hair removal device of claim 2, wherein the main body is snapped to the optical hair removal assembly by the detachable assembly;
wherein the detachable assembly comprises a snapping body and a snapping base for snap-fit, and the snapping body is provided in one of the main body and the optical hair removal assembly; and
the snapping base is provided in another one of the main body and the optical hair removal assembly.

4. The optical hair removal device according to claim 3, wherein the snapping body is protruded at a rear end of the housing, and the snapping body comprises a convex extension and a snapping portion connected to each other, wherein an angle is formed between the snapping portion and the convex extension, and the convex extension is connected to the rear end of the housing; and
the snapping base is provided at a front end of the main body, the snapping base is provided with a groove with an opening forward, and the snapping portion is snapped in the groove when the optical hair removal assembly is docked with the main body.

5. The optical hair removal device according to claim 3, wherein the detachable assembly further comprises a limitation member, the limitation member is provided in one of the main body and the optical hair removal assembly, and another one of the main body and the optical hair removal assembly is provided with a limitation groove corresponding one-in-one to the limitation member; and
the limitation member is movably accessed into the limitation groove, to lock the optical hair removal assembly and the main body.

6. The optical hair removal device according to claim 5, wherein the limitation member is provided at a front end of the main body, wherein the limitation member comprises a pressing portion and a limitation portion, and the pressing portion is protruded at one of sides of the main body, and the limitation portion is protruded at a front end of the main body;
the limitation groove is provided at a rear end of the optical hair removal assembly; and
the limitation member is movably accessed into the limitation groove, to lock the optical hair removal assembly and the main body.

7. The optical hair removal device according to claim 2, wherein the detachable assembly is a connection cable, and the connection cable is detachably connected to at least one of the optical hair removal assembly and the main body; and
an end of the connection cable is electrically connected to the optical hair removal assembly, another end of the connection cable is electrically connected to the main body, and a control main board of the main body is electrically connected to the optical hair removal assembly by the connection cable.

8. The optical hair removal device according to claim 2, wherein the main body is electrically connected to the optical output assembly by a converting device, and the converting device comprises a converting wire and at least one converter;
each converter is detachably and electrically connected to one of the main body and the optical hair removal assembly by the detachable assembly, an end of the converting wire is physically and electrically connected to the converter, and another end of the converting wire is physically and electrically connected to another one of the main body and the optical hair removal assembly.

9. The optical hair removal device according to claim 8, wherein the converter is snapped to the main body or the optical hair removal assembly; and
a set of electrical connectors adapted and electrically connected to each other are provided at surfaces where the converter is docked with the main body or the optical hair removal assembly, respectively.

10. The optical hair removal device according to claim 9, wherein the converting device comprises two converters provided at two opposite ends of the converting wire, and the two converters are a first converter and a second converter, respectively;
the first converter is snapped to the main body by the detachable assembly, a first set of electrical connectors adapted and electrically connected to each other are provided at surfaces where the first converter is docked with the main body, respectively; and
the second converter is snapped to the optical hair removal assembly by the detachable assembly, and a second set of electrical connectors adapted and electrically connected to each other are provided at surfaces where the second converter is docked with the optical hair removal assembly.

11. The optical hair removal device according to claim 9, wherein the converting device comprises a converting wire and a converter, an end of the converting wire is physically and electrically connected to the converter, and another end of the converting wire is physically and electrically connected to the main body, wherein the converter is snapped to the optical hair removal assembly by the detachable assembly; and
a set of electrical connectors adapted and electrically connected to each other are provided at surfaces where the converter is docked with the optical hair removal assembly.

12. The optical hair removal device according to claim 9, wherein the converting device comprises a converting wire and a converter, an end of the converting wire is physically and electrically connected to the converter, and another end of the converting wire is physically and electrically connected to the optical hair removal assembly, and the converter is snapped to the main body by the detachable assembly; and
a set of electrical connectors adapted and electrically connected to each other are provided at surfaces where the converter is docked with the main body.

13. The optical hair removal device according to claim 1, wherein the optical hair removal device comprises a plug-in connection terminal set;
wherein the plug-in connection terminal set comprises a first connection terminal and a second connection terminal, wherein the first connection terminal is provided at a rear end of the optical hair removal assembly, the second connection terminal is provided at a front end of the main body, and the first connection terminal is pluggable with the second connection terminal.

14. The optical hair removal device according to claim 1, wherein the hair removal device comprises a power transmission cable, an end of the power transmission cable is fixed to the optical hair removal assembly, and an electrical connection of the power transmission cable and the optical hair removal assembly is kept, another end of the power transmission cable is fixed to the main body, and an electrical connection of the power transmission cable and the main body is kept.

15. The optical hair removal device according to claim 1, further comprising a plurality of the main bodies, wherein each main body is provided with a battery inside, and the battery is electrically connected to the control main board.

16. The optical hair removal device according to claim 1, further comprising a plurality of the optical hair removal assemblies, wherein the plurality of the optical hair removal assemblies comprise a first optical hair removal assembly and a second optical hair removal assembly, a light outlet of the first optical hair removal assembly is a circular light outlet, and a light outlet of the second optical hair removal assembly is a square light outlet.

17. The optical hair removal device according to claim 1, wherein the optical hair removal device further comprises an adapter, the adapter is separated from the main body, and the adapter and the main body are electrically connected to the control main board by a wire.

18. The optical hair removal device according to claim 1, wherein the main body comprises a housing;
an end of the heat dissipation channel is an air inlet and another end of the heat dissipation channel is an air outlet, and the air inlet is located on a side of the housing and the air outlet is located on another side of the housing.

19. The optical hair removal device according to claim 1, wherein the light emitting assembly comprises a light source, a reflective cover, and a lens, wherein the light source is fixed on an inner side of the reflective cover, and is a pulsed lamp or a laser light source;
the housing is also provided with a positioning bracket, and the reflective cover is fixed to the positioning bracket; and
the lens is provided at a front of the reflective cover for converging light emitted by the light source.
